(19) Europäisches Patentamt
European Patent Office
Office européen des brevets

(11) EP 0 891 324 B1

(12) EUROPEAN PATENT SPECIFICATION

(45) Date of publication and mention of the grant of the patent:
**12.09.2001 Bulletin 2001/37**

(21) Application number: **97917051.1**

(22) Date of filing: **27.03.1997**

(51) Int Cl.[7]: **C07C 253/30**, C07F 9/6574, B01J 31/18, B01J 21/18

(86) International application number:
**PCT/US97/04997**

(87) International publication number:
**WO 97/36856 (09.10.1997 Gazette 1997/43)**

(54) **CATALYZED VAPOR-PHASE ISOMERIZATION OF NONCONJUGATED 2-ALKYL-3-MONOALKENENITRILES**

KATALYSIERTE GASPHASENISOMERISIERUNG VON NICHT KONJUGIERTEN 2-ALKYL-3-MONOALKENNITRILEN

ISOMERISATION EN PHASE VAPEUR CATALYSEE DE 2-ALKYL-3-MONOALCENENITRILES NON CONJUGUES

(84) Designated Contracting States:
**DK FR GB IT NL**

(30) Priority: **02.04.1996 US 14534 P**

(43) Date of publication of application:
**20.01.1999 Bulletin 1999/03**

(73) Proprietor: **E.I. DU PONT DE NEMOURS AND COMPANY**
**Wilmington Delaware 19898 (US)**

(72) Inventors:
- **DRULINER, Joe, Douglas**
  **Newark, DE 19711 (US)**
- **HERRON, Norman**
  **Newark, DE 19711 (US)**

(74) Representative: **Jones, Alan John et al**
**CARPMAELS & RANSFORD**
**43 Bloomsbury Square**
**London, WC1A 2RA (GB)**

(56) References cited:
**WO-A-95/10481** **US-A- 5 440 067**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 891 324 B1

## Description

This invention generally relates a vapor-phase process for the isomerization of 2-alkyl-3-monoalkenenitriles to produce 3- and/or 4-monoalkene linear nitriles by using zero-valent nickel and a multidentate phosphite ligand as catalysts and, more particularly to the use of specialized catalyst supports and treatments thereof.

### BACKGROUND OF THE INVENTION

Hydrocyanation of butadiene to form pentenenitrile (PN) is known in the art, e.g., U.S. Patent 3,766,237. The predominant pentenenitriles so formed, e.g., 3-pentenenitrile, 4-pentenenitrile and 2-methyl-3-butenenitrile, are further subjected to hydrocyanation and/or isomerization to form adiponitrile (ADN), a commercially important material in the synthesis of nylon.

In the liquid phase, 2-methyl-3-butenenitrile can be efficiently isomerized to 3- and/or 4-pentenenitrile, in the presence of zero-valent nickel catalysts, which products are easily further hydrocyanated to form adiponitrile. U.S. Patent 5,440,067 describes a process for the vapor-phase isomerization of an acylic, aliphatic, nonconjugated 2-alkyl-3-monoalkenenitrile, preferably 2-methyl-3-butenenitrile, said process comprising contacting the starting nitrile, at a temperature within the range of about 135°C to about 170°C, with a supported catalyst composition comprising zero-valent nickel and at least one bidentate phosphite ligand. Typically, in accordance with U.S. Patent 5,440,067, the zero-valent nickel catalysts are dispersed on silica, alumina or carbon supports in a conventional manner.

While the vapor-phase isomerization process described in U.S. Patent 5,440,067 offers many advantages over liquid phase process for optimum commercial use, the life of the catalyst needs to be prolonged. The invention described herein provides for longer catalyst life in the vapor-phase isomerization of nonconjugated 2-alkyl-3-monoalkenenitriles by the selection of catalysts, catalyst supports and catalyst support pre-treatments. Other objects and advantages of the present invention will become apparent to those skilled in the art upon reference to the detailed description which hereinafter follows.

### SUMMARY OF THE INVENTION

The present invention provides a process for the vapor-phase isomerization of an acyclic, aliphatic, nonconjugated 2-alkyl-3-monoalkenenitrile, preferably 2-methyl-3-butenenitrile, said process comprising contacting the starting nitrile, at a temperature within the range of 135°C to 300°C, with a supported catalyst composition comprising zero-valent nickel and at least one multidentate phosphite ligand selected from the group consisting of Formula I and Formula II:

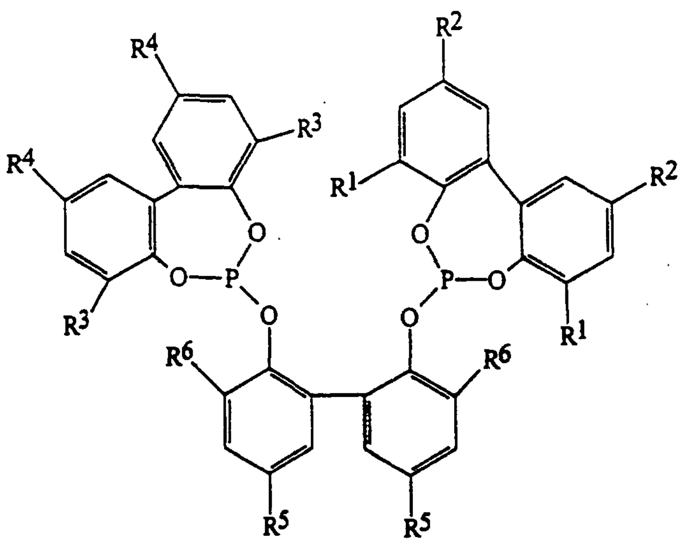

Formula I

wherein

each $R^1$, $R^2$, $R^3$, $R^4$, $R^5$, and $R^6$ are independently, H, a branched or straight chain alkyl of up to 12 carbon atoms,

or $OR^7$, wherein $R^7$ is a $C_1$ to $C_{12}$ alkyl.

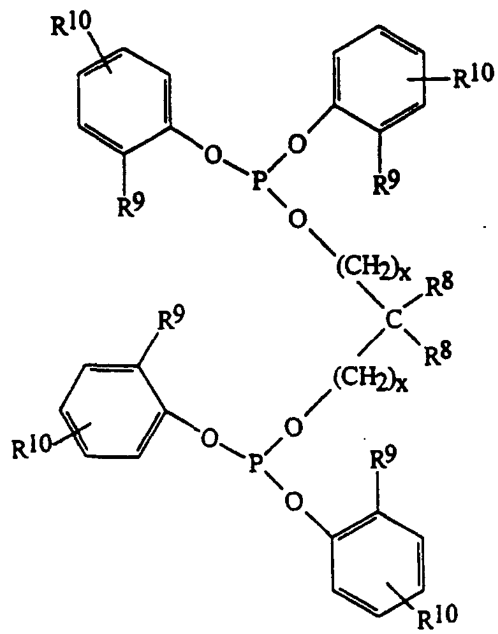

Formula II

wherein

each $R^8$ is, independently, H, a primary, secondary or tertiary hydrocarbyl of 1 to 12 carbon atoms, or B, where B is a substituent of the formula

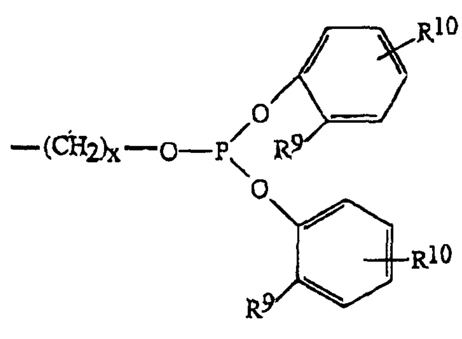

wherein x is an integer from 1 to 12,

and

each $R^9$ and $R^{10}$ are independently, H, $OR^{11}$ wherein $R^{11}$ is a $C_1$ to $C_{12}$ alkyl or a primary, secondary or tertiary hydrocarbyl of 3 to 12 carbon atoms and wherein $R^{10}$ can be ortho, meta or para to the oxygen; and wherein x is an integer from 1 to 12; and wherein the catalyst support is selected form the group consisting of heat-treated carbon, heated to a temperature of at least 600°C in an inert atmosphere for at least 2 hours, and an organic polymer; to produce nonconjugated, linear, acyclic 3-and/or 4-monoalkenenitriles.

The process may be carried out continuously or batchwise.

In the above definitions for Formula I and Formula II, "secondary" and "tertiary" refer to the carbon atom attached to the aromatic ring. In addition, for purposes of the present disclosure and claims, the terms "alkenenitrile", "pentenenitrile", and "butenenitrile" are intended to mean, respectively, a cyanoalkene in which the carbon atom of the cyano group is the first carbon; a cyanobutene; and a cyanopropene.

The present invention provides a catalyzed vapor-phase process which is rapid, selective, efficient and stable in the isomerization of nonconjugated 2-alkyl-3-monoalkenenitriles. Advantages of this vapor-phase process include

the elimination of certain solvents used in most liquid phase processes, such as reaction diluents or product extractants- Furthermore, in the vapor-phase process, the catalyst is utilized as a stationary solid phase, which can reduce catalyst synthesis, recovery, recycle and by-product waste disposal costs. Such advantages can eliminate the need for equipment associated with liquid phase processes and provide further cost savings. Utilization of particular multidentate ligands and specially selected and/or treated catalyst supports can greatly extend catalyst life providing expediency for commercial application and further savings.

DETAILED DESCRIPTION OF THE PREFERRED EMBODIMENTS

When the starting nitrile is 2-methyl-3-butenenitrile (2M3BN), the vapor-phase isomerization process of this invention produces a mixture of pentenenitriles consisting essentially of 3-pentenenitrile (3PN) and 4-pentenenitrile (4PN).

Zero-valent nickel is known in the art and can be made in a number of ways. Most common zero-valent nickel species, which can be used to form catalytic compositions useful in the present invention, are derived from Ni(O) complexes containing o-tritolylphosphite, p-tritolylphosphite, cyclooctadiene, and ethylene. Ni(O) can also be prepared by reduction of Ni(II) compounds with molecular hydrogen, or other reducing agents, in the presence of appropriate ligands hereinafter where $Ni(NO_3)_2$ is reduced by $H_2$ to provide Ni(O) on silica gel. Moreover, Ni(O) complexes containing multidentate ligands can be prepared from reduction of Ni(II) compounds and Ni metal and a multidentate ligand. Other zero-valent nickel species known to those skilled in the art can be successfully used as well.

The actual catalyst is a complex of zero-valent nickel with the multidentate phosphite ligand, which is formed when those two materials are combined. An effective catalyst requires at least one mole of multidentate phosphite ligand for one gram-atom of zero-valent nickel.

The catalyst compositions of Formula II can be supported on a carrier of specially treated carbon or polymeric materials discussed below. Commonly used techniques for treatment of supports with metal catalysts can be found in B. C. Gates, Heterogeneous Catalysis. Vol. 2, pp. 1-29, Ed. B. L. Shapiro, Texas A & M University Press, College Station, Texas, 1984. Alternately, a given support can be prepared with a uniformly dispersed coating of zero-valent nickel metal, and then can be treated with the desired multidentate phosphite ligand.

Typically, in accordance with this invention, the zero-valent nickel catalysts are dispersed on the selected catalyst supports at concentrations sufficient to produce a supported catalyst composition containing 0.3 wt. % to 1.0 wt. % Ni.

The catalyst compositions of Formula I are supported on catalyst supports selected from heat-treated carbon or organic polymer supports. The carbon utilized can be any form of commercially available particulate carbon or specially prepared carbon soot, but the carbon must be heated in an inert atmosphere to at least 600°C, and preferably to at least 800°C, for at least 2 hours, and preferably at least 4 hours. prior to contact with the catalyst and subsequent use. After the carbon has been heat-treated and cooled, the catalyst can be applied thereto by dispersion of the catalyst in an organic liquid, sich as toluene or tetrahydrofuran, and contacting this dispersion with the carbon. The organic polymer supports of choice are those with thermal stabilities greater than 200°C, for example, polydivinylbenzene / polyacrvlonitriie and cross-linked polystyrene and copolymers or blends thereof. In the case of organic polymer supports the catalyst of choice can be dispersed therewith in the same manner as utilized for carbon supports.

The catalyst compositions are then loaded into tubular reactors, and gaseous 2-alkyl-3-monoalkenenitrile, e. g., 2-methyl-3-butenenitrile, is passed continuously over the solid catalysts at temperatures sufficiently high to maintain both the starting nitrile and the isomerization products in the vapor-phase. The temperature range is generally from 135°C to 300°C and preferably from 145°C to 200°C. The temperature must be high enough to keep all the organic materials volatilized, but low enough to prevent deterioration of the catalyst. The preferred temperature is dependent to a certain extent on the particular catalyst employed, the particular 2-alkyl-3-monoalkenenitrile being used, and the desired reaction rate. Operating pressure is not particularly critical and can conveniently be from about 1 to about 10 atmospheres (101.3 to 1013 kPa). No economic benefit can be expected above that range.

The 2-alkyl-3-monoalkenenitrile starting material can be delivered either as a neat vapor or as a vaporized solution in a solvent, such as acetonitrile or toluene. Under atmospheric pressure, using an additional feed of nitrogen or another inert gas as a carrier, temperatures of 140-160°C are typically used. Nitrogen is preferred because of its low cost. Gaseous oxygen, water vapor, or other gaseous substance which could react with either the catalyst or the starting 2-alkyl-3-monoalkenenitrile should be avoided. The isomerization products are liquid at room temperature and can be conveniently recovered by cooling.

The olefinic double bond in the 2-alkyl-3-monoalkenenitriles used as the starting materials in the process of this invention cannot be conjugated to the triple bond of the cyano group. Suitable starting 2-alkyl-3-monoalkenenitriles can also carry groups which do not attack the catalyst, for example another cyano group. Preferably, the starting 2-alkyl-3-monoalkenenitriles contain from 5 to 8 carbon atoms, excluding any additional substitution. 2-Methyl-3-butenenitrile is especially important in the production of adiponitrile. Other representative nitriles include 2-ethyl-3-butenenitrile and

2-propyl-3-butenenitrile.

The following Formulas III and IV illustrate suitable representative starting 2-alkyl-3-monoalkenenitriles. When the starting nitrile is 2-methyl-3-butenenitrile, the isomerization products are those shown in Formulas V and VI.

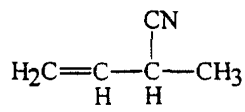

Formula III

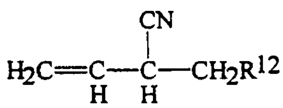

wherein

$R^{12}$ is H or a $C_1$ to $C_3$ alkyl.

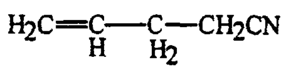

Formula V

and

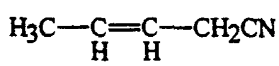

Formula VI

It will be recognized that Formula III is a special case of Formula IV, where $R^{12}$ is hydrogen.

The present isomerization process can be carried out, for example, by first charging a tubular reactor with a particular supported catalyst composition, preferably in an inert atmosphere, and then connecting it to a continuous feeding and recovery system while minimizing air contact by suitably purging all feed lines with a flow of an inert gas, such as nitrogen, argon, or helium. The reactor, equipped with a thermocouple, is then heated to the desired reaction temperature, either under a continuous flow of inert gas, or with both the inlet and the outlet closed, while the inert gas is flowing through a bypass. The starting 2-alkyl-3-monoalkenenitrile can be fed either neat or dissolved in a suitable solvent, such as, for example, acetonitrile or toluene. The starting nitrile and any solvent are passed through a portion of the feed line heated to the reaction temperature to ensure complete vaporization. The gaseous product mixture exiting through the reactor outlet can be passed, if desired, through a heated gas sampling loop of a gas chromatograph for monitoring the progress of the reaction. Alternatively, the gaseous effluent can be cooled to 0°C-25°C in order to recover the products as liquids. The mole ratio of unsaturated compound to catalyst, per hour of continuous feed, normally can be varied from about 5:1/hour to 100:1/hour.

The isomerization reaction is preferably carried out without a solvent. If any solvent is used, it should be gaseous at the reaction temperature and pressure and inert towards the starting nitrile, the reaction products, and the catalyst. Typical solvents include hydrocarbons such as hexane, benzene, toluene, and xylene, or nitriles such as acetonitrile.

<u>EXAMPLES</u>

The following non-limiting, representative examples illustrate the present invention. All proportions and percentages are by weight unless otherwise indicated. In the examples, Ligand "A" is the ligand of Formula I, where each $R^5$ is $OCH_3$, and each $R^6$ is t-butyl. Ligand PE-1 is the ligand of Formula II where x is 1 and $R^9$ is isopropyl and $R^{10}$ is

H. Ligand PE-2 is the ligand of Formula II where x is 1 and $R^8$ is B, $R^9$ is t-butyl and $R^{10}$ is para-t-butyl. Ligand PE-3 is the ligand of Formula II where x is 1 and $R^9$ is isopropyl, $R^{10}$ is meta-$CH_3$ and $R^8$ is B.

COMPARATIVE EXAMPLE 1

Synthesis of Silica-supported $Ni(OTTP)_2CH_2=CH_2$ + PE-1 where OTTP is o-tritolyl phosphite and the Ligand "PE-1" is the ligand of Formula II, where X = 1, each $R^8$ = B, $R^9$ is isopropyl and $R^{10}$ is H

A 2 g sample of Johnson Mathey Co. silica (300 $m^2/g$, 8/12 mesh) was dried under flowing $N_2$ at 200°C for 2 hours, then cooled to ambient temperature in a $N_2$-filled glove box. Next, 0.19 g (0.125 mmole) Ligand PE-1, 0.10 g (0.125 mmole) $Ni(OTTP)_2CH_2=CH_2$ and 10 mL dry THF were combined with the the dried silica and the mixture was stirred for 15 minutes. Solvent THF was evaporated under vacuum to provide the silica-supported Ni(0) catalyst used for Example 12 in Table 1.

COMPARATIVE EXAMPLE 2

Synthesis of Silica-supported $Ni(OTTP)_2CH_2=CH_2$ + PE-2 where OTTP is o-tritolyl phosphite and the Ligand "PE-2" is the ligand of Formula II, where X = 1, each $R^8$ = B, each $R^9$ is t-butyl and each $R^{10}$ is para-t-butyl

A 1 g sample of Johnson Mathey Co. silica (300 $m^2/g$, 8/12 mesh), dried as in Example 1, was combined with 0.119 g (0.0625 mmole) Ligand PE-2, 0.05 g (0.0625 mmole) $Ni(OTTP)_2CH_2=CH_2$ and 10 mL dry THF and the mixture was stirred for 15 minutes. Solvent THF was evaporated under vacuum to provide the silica-supported Ni(0) catalyst used for Example 13 in Table 1.

COMPARATIVE EXAMPLE 3

Synthesis of Silica-supported $Ni(OTTP)_2CH_2=CH_2$ + PE-3 where OTTP is o-tritolyl phosphite and the Ligand "PE-3" is the ligand of Formula II. where X is 1, each $R^8$ = B, $R^9$ isopropyl and $R^{10}$ is meta-methyl

A 1 g sample of Johnson Mathey Co. silica (300 $m^2/g$, 8/12 mesh), dried as in Example 1, was combined with 0.19 g (0.125 mmole) Ligand PE-3, 0.10 g (0.125 mmole) $Ni(OTTP)_2CH_2=CH_2$ and 10 mL dry THF and the mixture was stirred for 15 minutes. Solvent THF was evaporated under vacuum to provide the silica-supported Ni(0) catalyst used for Example 14 in Table 1.

EXAMPLE 4

Synthesis of polydivinylbenzene/polyacrylonitrile-supported Ni(0) (Ligand "A") $CH_2=CH_2$, where the Ligand "A" is of Formula I, where each R1, $R^2$, $R^3$, and $R^4$ is H, $R^5$ is $OCH_3$ and each $R^6$ is t-butyl

A 10 g sample of Johns Manville Chromasorb 104 (60/80 mesh), a commercially available polydivinylbenzene/polyacrylonitrile, was dried in flowing $N_2$ at 150°C for 1 hour, then cooled to ambient temperature in a $N_2$-filled glove box. Next, 2 g of the dried Chromasorb 104 was combined with 0.11 g (0.125 mmole) Ni(0) (Ligand "A") $CH_2=CH_2$ and 10 mL dry THF and the mixture was stirred for 10 minutes. Solvent THF was evaporated under vacuum to provide the Chromasorb-104-supported Ni(0) catalyst used for Example 15 in Table 1.

EXAMPLE 5

Synthesis of polydivinylbenzene/polyacrylonitrile-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

A 1 g sample of Chromasorb 104, dried as in Example 4, was combined with 0.10 g (0.24 mmole) Ni(0) (Ligand "A")$_2$ and 10 mL dry (THF) and was stirred for 15 minutes. Solvent THF was evaporated under vacuum to provide the Chromasorb 104-supported Ni(0) catalyst used for Example 16 in Table 1.

EXAMPLE 6

Synthesis of cross-linked polystyrene-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 5 was followed except that Johns Manville Chromasorb 103 (60-80 mesh), a commercially available cross-linked polystyrene, was used as the catalyst support for Example 17 in Table 1.

EXAMPLE 7

Synthesis of a heat-treated carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 5 was followed except that heat-treated carbon soot was used as the catalyst support. The carbon soot was prepared by vaporizing a solid carbon rod, using an electric arc , in an inert atmosphere. Carbon soot was produced by an electric arc method in a vacuum chamber. Two 1/8" graphite rods were used as electrodes. The vacuum chamber was first evacuated and then back-filled with 150 torr of helium. Electrical currents of 120 amp at ~20 volts were passed through the rods to vaporize the carbon which redeposits on the walls of the chamber to form ultrafine carbon soot. The initially generated soot contained fullerenes which were removed by extraction with toluene. The remaining carbon soot was used for catalysis study.

The soot was then heated to 850°C in a $CO_2$ atmosphere for 5 hours. After cooling, the catalyst was dispersed onto the heat-treated carbon soot using (THF) for use in Example 18 in Table 1.

COMPARATIVE EXAMPLE 7A

Synthesis of a carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 7 was followed except that the carbon soot was not heat-treated prior to use for the catalyst preparation used in Comparative Example 18A in Table 1.

COMPARATIVE EXAMPLE 7B

Synthesis of a carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 7 was followed except that the carbon soot was not heat-treated prior to use, and toluene was used to disperse the catalyst onto the carbon soot support used for Comparative Example 18B in Table 1.

EXAMPLE 8

Synthesis of a heat-treated carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

A 5 g sample of commercially available Calsicat hydrochloric acid-extracted carbon (1500 $m^2$/g, 4 x 8 mesh) was heated to 850°C in a $CO_2$ atmosphere for 5 hours. After cooling, 1 g of the heated carbon was combined with 0.10 g (0.24 mmole) Ni(0) (Ligand "A")$_2$ and 10 mL dry toluene and was stirred for 15 minutes. Solvent toluene was evaporated under vacuum to provide the heat-treated carbon-supported Ni(0) catalyst used for Example 19 in Table 1.

EXAMPLE 9

Synthesis of a heat-treated carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 8 was followed except that (THF) was used instead of toluene to disperse the catalyst onto the heat-treated carbon support used for Example 20 in Table 1.

EXAMPLE 10

Synthesis of a heat-treated carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

The procedure of Example 9 was followed except that the commercially available carbon was heated at 850°C for 5 hours under an atmosphere of helium instead of $CO_2$ for use in preparation of the catalyst used in Example 21 in Table 1.

EXAMPLE 11

Synthesis of a heat-treated carbon-supported Ni(0) (Ligand "A")$_2$, where the Ligand "A" is of Formula I, where each $R^1$, $R^2$, $R^3$, and $R^4$ is H, $R^5$ is $OCH_3$ and each $R^6$ is t-butyl

A 3 g sample of commercially available EM Co. carbon (1050 $m^2$/g, 20-35 mesh) was heated to 850°C in a helium atmosphere for 5 hours. After cooling, 3 g of the heated carbon was combined with 0.30 g (0.72 mmole) Ni(0) [Ligand "A"]$_2$ and 30 mL dry (THF) and was stirred for 15 minutes. Solvent THF was evaporated under vacuum to provide the heat-treated carbon-supported Ni(0) catalyst used for Example 22 in Table 1.

EXAMPLES 12-22

Vapor Phase Isomerization of 2-methyl-3-butenenitrile (2M3BN)

In a nitrogen-filled glove box, a plug of glass wool was placed in the bottom of an empty 0.25-inch (0.64 cm) diameter x 15-inch (38.1 cm) long stainless steel tubular reactor. Then, the amount and type of Ni(0) catalyst shown in Table 1 was introduced into the reactor. In all the examples using a catalyst composition comprising a bidentate phosphite ligand, the ligand was Ligand "A". The reactor was then connected to a feed and product recovery system purged with nitrogen. The starting nitrile was fed either as a 8.2-10.6% solution of 2M3BN in acetonitrile or as neat 2M3BN. Attached to the exit side of the reactor was a jacketed receiver with circulating ethylene glycol cooled to 0°C. Gas Chromatograph (GC) analyses were done on a 30 m DB-23 capillary column of a 0.32 mm internal diameter, supplied by J&W Scientific, Folsom, California. The nitrogen, 2M3BN, and acetonitrile feed streams were preheated to 165°C to ensure complete vaporization. The reactor was heated in a split tube furnace to the temperature shown in Table 1. Product samples were collected, approximately every hour.

Table I summarizes the experimental conditions as well as the results. By "% conv." is meant

$$100 \times \frac{(\text{measured GC area \% for 3PN + 4PN})}{(\text{measured GC area \% for 3PN + 4PN + 2M3BN})}$$

The starting 2M3BN, used as feed, contained small amounts of 3PN and 4PN, corresponding to about a 1.5% conversion, which is included in the data reported below. The starting 2M3BN also contained from about 15% to 30% by weight, based on the total weight of the starting 2M3BN, of cis and trans 2M2BN and cis 2PN. Comparative Examples 12, 13 and 14 show that the ligands of Formula II also produce long-lived active catalysts on a conventional silica support. Examples 15 and 16 show that the polydivinylbenzene/polyacrylonitrile support renders long life to the catalysts of Formula I. The cross-linked polystyrene support of Example 17 produced an undesirable amount of conjugated product, but this is may be due to impurities in the polystyrene material. Example 18 shows that heat-treated carbon soot makes an excellent support relative to carbon soot which is not heat treated, as shown in Comparative Examples 18A and 18B. Examples 19, 20 and 21 show the positive effect of heat treatment on acid washed carbon. Example 22 shows the positive effects of heat treatment on commercially available carbon.

In the Table, "A" stands for Ligand "A"; "PE-1" for Ligand "PE-1"; "PE-2" for Ligand "PE-2"; "PE-3" for Ligand "PE-3"; and "OTTP" stands for o-tritolyl phosphite.

**TABLE 1**

**Vapor Phase Isomerization of 2M3BN to 3PN and 4PN**

| Ex. | Catalyst (Support) | Catalyst g, % Ni | 2M3BN Feed Mmole/hr | $N_2$ Feed mL/min | 2M3BN/Ni mole/gramatom | Temp. °C | Elapsed Time hr. | (3PN) GC Area % | (4PN) GC Area % | % Conv. |
|---|---|---|---|---|---|---|---|---|---|---|
| 12 (comparative) | $Ni[OTTP]_2CH_2{=}CH_2$ + PE-1 | 1.05,0.32 | 1.7 | 10 | 28.2 | 145 | 1 | 57.7 | 2.7 | 81.9 |
| | | | | | | | 5 | 68.3 | 2.0 | 90.5 |
| | (Silica) | | | | | | 12 | 65.1 | 0.6 | 85.4 |
| | Comparative Ex. 1 | | | | | | 18 | 66.1 | 0.6 | 83.8 |
| | | | | | | | 24 | 45.2 | 0.3 | 57.3 |
| 13 (comparative) | $Ni[OTTP]_2CH_2{=}CH_2$ + PE-2 | 1.00,0.29 | 1.2 | 10 | 18.5 | 145 | 1 | 48.6 | 1.4 | 91.4 |
| | | | | | | | 5 | 42.8 | 0.9 | 90.2 |
| | (Silica) | | | | | | 10 | 51.3 | 0.9 | 91.3 |
| | Comparative Ex. 2 | | | | | | 15 | 49.3 | 0.7 | 90.4 |
| | | | | | | | 20 | 51.6 | 1.3 | 92.1 |

| Ex. | Catalyst (Support) | Catalyst g, % Ni | 2M3BN Feed Mmole/hr | $N_2$ Feed mL/min | 2M3BN/Ni mole/gramatom | Temp. °C | Elapsed Time hr. | (3PN) GC Area % | (4PN) GC Area % | % Conv. |
|---|---|---|---|---|---|---|---|---|---|---|
| 14 (comparative) | Ni[OTTP]$_2$CH$_2$=CH$_2$ + PE-3 (Silica) Comparative Ex. 3 | 1.02,0.31 | 1.7 | 10 | 28.2 | 145 | 1 | 67.4 | 3.7 | 91.9 |
| | | | | | | | 3 | 69.0 | 3.4 | 92.1 |
| | | | | | | | 6 | 71.8 | 2.8 | 92.4 |
| | | | | | | | 9 | 71.7 | 3.9 | 92.8 |
| 15 | Ni["A"]CH$_2$=CH$_2$ | 1.04,0.34 | 1.7 | 10 | 26.2 | 145 | 1 | 74.9 | -- | 90.4 |
| | | | | | | | 24 | 78.0 | -- | 91.2 |
| | (Chromasorb 104) Ex. 4 | | | | | | 48 | 77.1 | -- | 90.9 |
| | | | | | | | 72 | 73.0 | -- | 88.9 |

| Ex. | Catalyst (Support) | Catalyst g, % Ni | 2M3BN Feed Mmole/hr | $N_2$ Feed mL/min | 2M3BN/Ni mole/gramatom | Temp. °C | Elapsed Time hr. | (3PN) GC Area % | (4PN) GC Area % | % Conv. |
|---|---|---|---|---|---|---|---|---|---|---|
| 16 | Ni["A"]$_2$ | 0.95,0.32 | 1.7 | 5 | 31.4 | 145 | 1 | 49.1 | -- | 90.5 |
| | | | | | | | 23.5 | 54.8 | -- | 89.3 |
| | (Chromasorb 104) Ex. 5 | | | | | | 47.5 | 74.2 | -- | 92.2 |
| | | | | | | | 71.5 | 71.9 | -- | 90.3 |
| | | | | | | | 92.5 | 69.5 | -- | 88.1 |
| 17 | Ni["A"]$_2$ | 1.01,0.32 | 1.7 | 5 | 30.8 | 145 | 1 | 14.5 | -- | 94.5 |
| | | | | | | | 5 | 8.1 | -- | 97.8 |
| | (Chromasorb 103) | | | | | | 9 | 5.4 | -- | 96.9 |
| | Ex. 6 | | | | | | 21 | 4.6 | -- | 97.2 |
| 18 | Ni["A"]$_2$ | 0.92,0.32 | 1.8 | 10 | 34.3 | 145 | 1 | 29.5 | 0.2 | 57.5 |
| | | | | | | | 10 | 60.1 | 1.0 | 75.3 |
| | (Carbon, heated) | | | | | | 20 | 64.8 | 1.5 | 80.0 |
| | Ex. 7 | | | | | | 30 | 67.3 | 1.6 | 83.2 |
| | | | | | | | 37 | 66.7 | 1.8 | 82.7 |

| Ex. | Catalyst (Support) | Catalyst g, % Ni | 2M3BN Feed Mmole/hr | $N_2$ Feed mL/min | 2M3BN/Ni mole/gramatom | Temp. °C | Elapsed Time hr. | (3PN) GC Area % | (4PN) GC Area % | % Conv. |
|---|---|---|---|---|---|---|---|---|---|---|
| 19 | Ni["A"]$_2$ | 1.08,0.32 | 1.7 | 5 | 28.2 | 145 | 2 | 37.3 | 0.3 | 84.4 |
| | | | | | | | 11 | 62.1 | 2.0 | 80.9 |
| | | | | | | | 20 | 50.5 | 1.0 | 65.2 |
| | (Carbon, heated) | | | | | | 26 | 45.0 | 0.6 | 56.5 |
| | Ex. 8 | | | | | | | | | |
| 18A | Ni["A"]$_2$ | 0.95,0.32 | 1.7 | 5 | 32.1 | 145 | 1 | 16.2 | -- | 89.3 |
| | (comparative) | | | | | | 11 | 13.9 | -- | 72.1 |
| | (Carbon, unheated) | | | | | | 23 | 13.4 | -- | 46.6 |
| | Ex. 7A | | | | | | 32 | 18.2 | -- | 44.3 |
| 18B | Ni["A"]$_2$ | 1.02,0.32 | 1.8 | 5 | 31.9 | 145 | 1 | 6.4 | -- | 13.6 |
| | (comparative) | | | | | | 1.5 | 4.0 | -- | 8.2 |
| | (Carbon, unheated) | | | | | | 2.25 | 4.7 | -- | 10.4 |
| | Ex. 7B | | | | | | 4 | 5.9 | -- | 12 |

| Ex. | Catalyst (Support) | Catalyst g, % Ni | 2M3BN Feed Mmole/hr | $N_2$ Feed mL/min | 2M3BN/Ni mole/gramatom | Temp. °C | Elapsed Time hr. | (3PN) GC Area % | (4PN) GC Area % | % Conv. |
|---|---|---|---|---|---|---|---|---|---|---|
| 20 | Ni["A"]$_2$ | 1.02,0.32 | 1.7 | 5 | 29.9 | 145 | 2 | 53.3 | 1.0 | 86.2 |
| | | | | | | | 12 | 73.7 | 6.7 | 94.7 |
| | (Carbon, heated) | | | | | | 24 | 63.9 | 2.2 | 87.2 |
| | Ex. 9 | | | | | | 36 | 65.3 | 1.1 | 79.9 |
| | | | | | | | 48 | 43.5 | 0.4 | 55.9 |
| 21 | Ni["A"]$_2$ | 1.01,0.32 | 1.7 | 10 | 30.2 | 145 | 2 | 59.5 | 0.3 | 74.5 |
| | | | | | | | 10 | 68.3 | 3.9 | 90.6 |
| | (Carbon, heated) Ex. 10 | | | | | | 16 | 75.4 | 2.9 | 92 |
| | | | | | | | 24 | 70.1 | 2.0 | 88.4 |
| | | | | | | | 36 | 62.8 | 0.9 | 76 |
| 22 | Ni["A"]$_2$ | 1.05,0.32 | 1.7 | 5 | 30 | 145 | 1.5 | 66.0 | 0.4 | 74.9 |
| | | | | | | | 10.6 | 70.8 | 1.4 | 80.1 |
| | (Carbon, heated) Ex. 11 | | | | | | 22.6 | 69.8 | 1.4 | 81.1 |
| | | | | | | | 31.6 | 73.4 | 1.6 | 84.3 |
| | | | | | | | 37.6 | 71.1 | 1.4 | 80.8 |

## Claims

**1.** A process for the vapor-phase isomerization of an acyclic, aliphatic, nonconjugated 2-alkyl-3-monoalkenenitrile comprising, contacting the starting nitrile, at a temperature within the range of from 135°C to 300°C, with a supported catalyst composition comprising zero-valent nickel and at least one multidentate phosphite ligand selected from the group consisting of compounds represented by Formulas I and II:

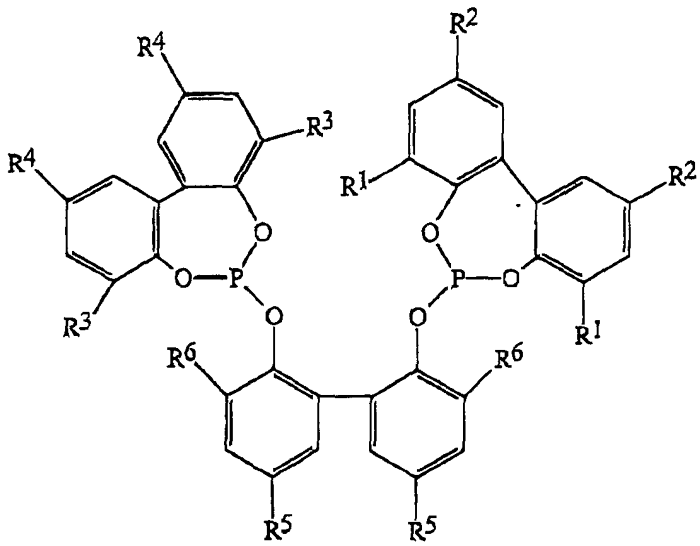

Formula I

wherein

each $R^1$, $R^2$, $R^3$, $R^4$, $R^5$, and $R^6$ are independently, H, a branched or straight chain alkyl of up to 12 carbon atoms, or $OR^7$, wherein $R^7$ is a $C_1$ to $C_{12}$ alkyl;

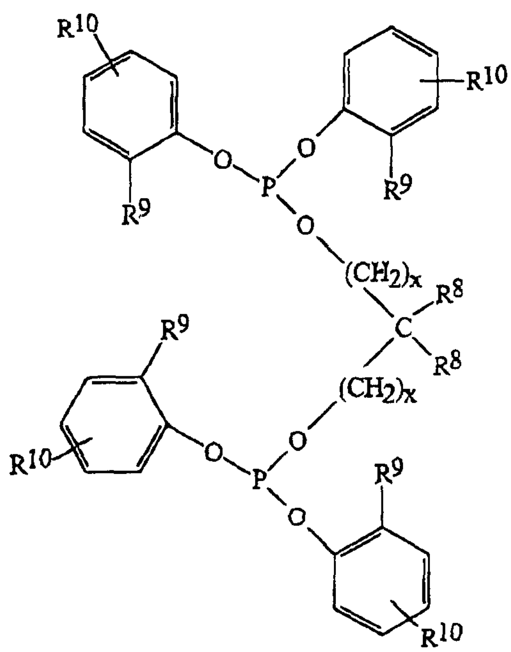

Formula II

wherein

each $R^8$ is, independently, H, a primary, secondary or tertiary hydrocarbyl of 1 to 12 carbon atoms, or B, where B is a substituent of the formula

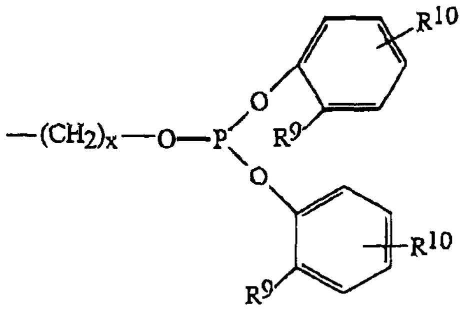

wherein x is an integer from 1 to 12;

and

each $R^9$ and $R^{10}$ are independently, H, $OR^{11}$ wherein $R^{11}$ is a $C_1$ to $C_{12}$ alkyl or a primary, secondary or tertiary hydrocarbyl of 3 to 12 carbon atoms and wherein $R^{10}$ can be ortho, meta or para to the oxygen; and wherein x is an integer from 1 to 12; and wherein the catalyst support is selected from the group consisting of heat-treated carbon, heated to a temperature of at least 600°C in an inert atmosphere for at least 2 hours, and an organic polymer; to produce nonconjugated, linear, acyclic 3-and/or 4-monoalkenenitriles.

**2.** The process of Claim 1 wherein the catalyst support is heated to at least 800°C.

**3.** The process of Claim 1 wherein the carbon suppon is heated for at least 4 hours.

**4.** The process of Claim 1 wherein the organic polymer suppon is selected from the group consisting of polydivinylbenzene/polyacrylonitrile, cross-linked polystyrene and copolymers and blends thereof

**5.** The process of Claim 1 wherein the starting 2-alkyl-3-monoalkenenitrile is a compound represented by the formula

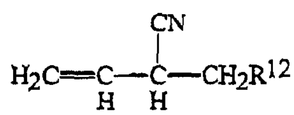

wherein

$R^{12}$ is H or a $C_1$ to $C_3$ alkyl.

**6.** The process of Claim 5 wherein the starting nitrile is 2-methyl-3-butenenitrile.

**7.** The process of Claim 1 wherein the temperature range is from 145°C to 200°C.

**Patentansprüche**

**1.** Verfahren für die Gasphasenisomerisierung eines acyclischen, aliphatischen, nichtkonjugierten 2-Alkyl-3-monoalkennitrils, umfassend das Inkontaktbringen des Ausgangsnitrils bei einer Temperatur innerhalb des Bereiches von 135°C bis 300°C mit einer geträgerten Katalysatorzusammensetzung, umfassend nullwertiges Nickel und mindestens einen mehrzähnigen Phosphitliganden, ausgewählt aus der Gruppe, bestehend aus Verbindungen, dargestellt durch die Formeln I und II:

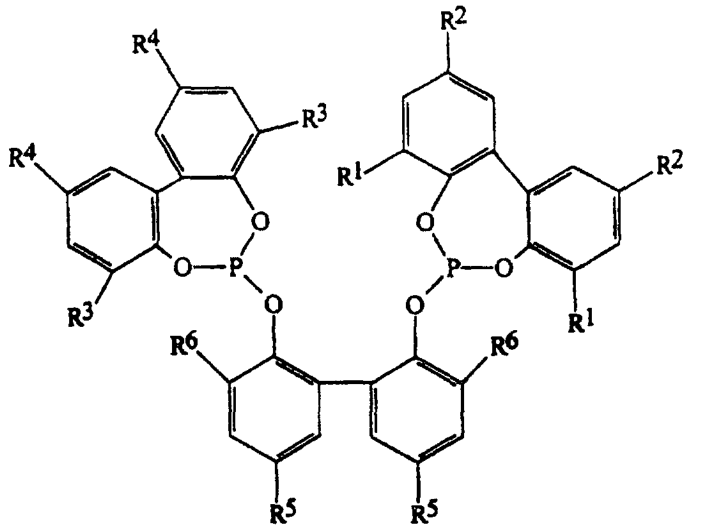

Formel I

wobei

jedes $R^1$, $R^2$, $R^3$, $R^4$, $R^5$ und $R^6$ unabhängig H, ein verzweigtes oder geradkettiges Alkyl mit bis zu 12 Kohlenstoffatomen oder $OR^7$ sind, wobei $R^7$ ein $C_1$- bis $C_{12}$-Alkyl ist;

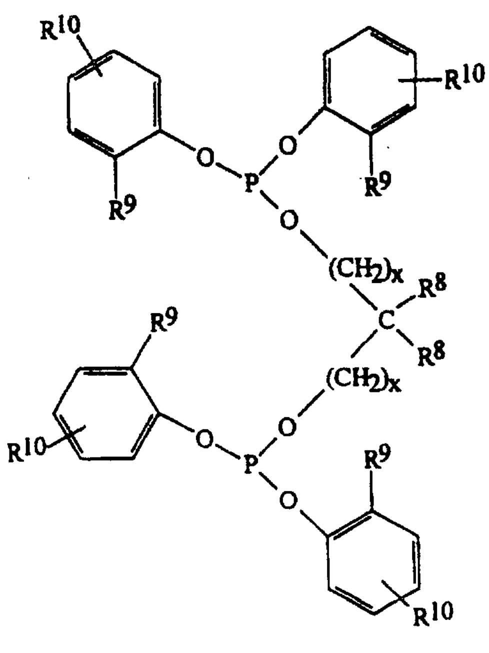

Formel II

wobei

jedes $R^8$ unabhängig H, ein primäres, sekundäres oder tertiäres Hydrocarbyl mit 1 bis 12 Kohlenstoffatomen oder B ist, wobei B ein Substituent der Formel

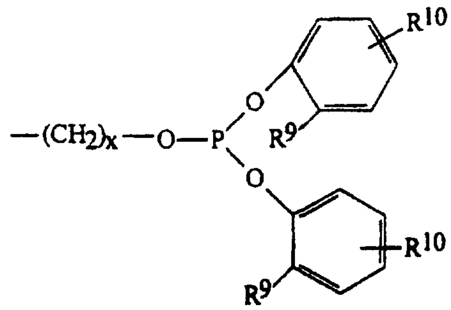

ist, wobei x eine ganze Zahl von 1 bis 12 ist;

und

jedes $R^9$ und $R^{10}$ unabhängig H, $OR^{11}$, wobei $R^{11}$ ein $C_1$- bis $C_{12}$-Alkyl ist, oder ein primäres, sekundäres oder tertiäres Hydrocarbyl mit 3 bis 12 Kohlenstoffatomen sind, und wobei $R^{10}$ ortho, meta oder para zu dem Sauerstoff stehen kann; und wobei x eine ganze Zahl von 1 bis 12 ist; und wobei der Katalysatorträger aus der Gruppe, bestehend aus wärmebehandeltem Kohlenstoff, erwärmt für mindestens 2 Stunden in einer inerten Atmosphäre auf eine Temperatur von mindestens 600°C, und einem organischen Polymer, ausgewählt ist; um nichtkonjugierte, lineare, acyclische 3- und/oder 4-Monoalkennitrile herzustellen.

**2.** Verfahren nach Anspruch 1, wobei der Katalysatorträger auf mindestens 800°C erwärmt wird.

**3.** Verfahren nach Anspruch 1, wobei der Katalysatorträger für mindestens 4 Stunden erwärmt wird.

**4.** Verfahren nach Anspruch 1, wobei der Träger in Form von organischem Polymer aus der Gruppe, bestehend aus Polydivinylbenzol/Polyacrylnitril, vernetztem Polystyrol und Copolymeren und Gemischen davon, ausgewählt ist.

**5.** Verfahren nach Anspruch 1, wobei das Ausgangs-2-Alkyl-3-monoalkennitril eine Verbindung, dargestellt durch die Formel

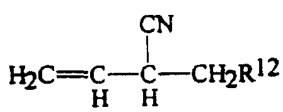

ist, wobei

$R^{12}$ H oder ein $C_1$- bis $C_3$-Alkyl ist.

**6.** Verfahren nach Anspruch 5, wobei das Ausgangsnitril 2-Methyl-3-butennitril ist.

**7.** Verfahren nach Anspruch 1, wobei der Temperaturbereich von 145°C bis 200°C beträgt.

**Revendications**

**1.** Procédé pour l'isomérisation en phase vapeur d'un 2-alkyl-3-monoalcènenitrile acyclique, aliphatique, non conjugué comprenant la mise en contact du nitrile de départ, à une température dans l'intervalle de 135°C à 300°C, avec une composition de catalyseur sur support comprenant un nickel de valence nulle et au moins un ligand de phosphite multidenté choisi dans le groupe constitué de composés représentés par les Formules I et II:

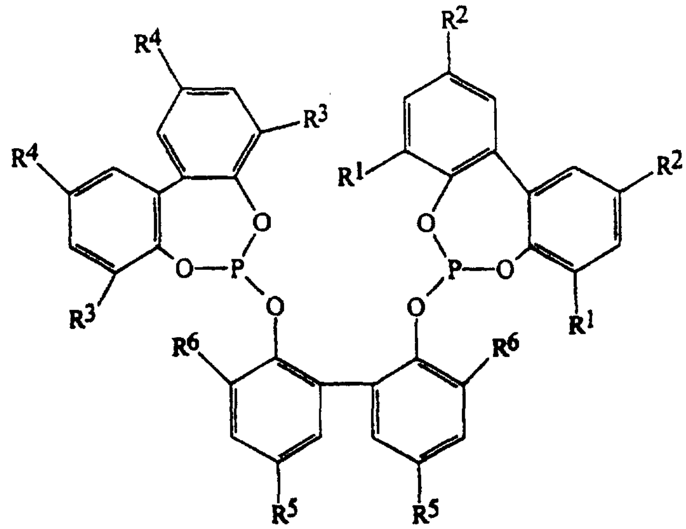

Formule I

dans laquelle:

chaque $R^1$, $R^2$, $R^3$, $R^4$, $R^5$ et $R^6$ sont indépendamment H, un groupe alkyle à chaîne ramifiée ou droite allant jusqu'à 12 atomes de carbone ou $OR^7$, dans laquelle $R^7$ est un groupe alkyle $C_1$ à $C_{12}$;

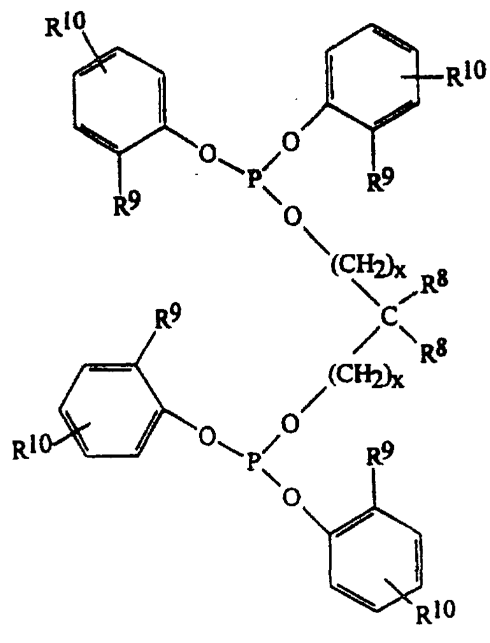

Formule II

dans laquelle:

chaque $R^8$ est, indépendamment, H, un groupe hydrocarbyle primaire, secondaire ou tertiaire de 1 à 12 atomes de carbone ou B, dans laquelle B est un substituant de la formule:

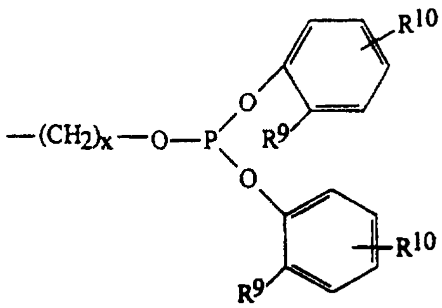

dans laquelle x est un nombre entier de 1 à 12;

et

chaque $R^9$ et $R^{10}$ sont indépendamment H, $OR^{11}$, dans laquelle $R^{11}$ est un groupe alkyle $C_1$ à $C_{12}$ ou un groupe hydrocarbyle primaire, secondaire ou tertiaire de 3 à 12 atomes de carbone et dans laquelle $R^{10}$ peut être situé en ortho, méta ou para par rapport à l'oxygène; et dans laquelle x est un nombre entier de 1 à 12; et dans laquelle le support de catalyseur est choisi dans le groupe constitué de carbone traité thermiquement, chauffé à une température d'au moins 600°C dans une atmosphère inerte pendant au moins 2 heures, et d'un polymère organique; pour produire des 3- et/ou 4-monoalcènenitriles non conjugués, linéaires, acycliques.

**2.** Procédé suivant la revendication 1, dans lequel le support de catalyseur est chauffé jusqu'à au moins 800°C.

**3.** Procédé suivant la revendication 1, dans lequel le support de carbone est chauffé pendant au moins 4 heures.

**4.** Procédé suivant la revendication 1, dans lequel le support de polymère organique est choisi dans le groupe constitué de polydivinylbenzène/polyacrylonitrile, de polystyrène réticulé et de copolymères et de mélanges de ceux-ci.

**5.** Procédé suivant la revendication 1, dans lequel le 2-alkyl-3-monoalcènenitrile de départ est un composé représenté par la formule:

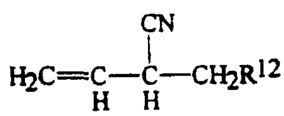

dans laquelle:

$R^{12}$ est H ou un groupe alkyle de $C_1$ à $C_3$.

**6.** Procédé suivant la revendication 5, dans lequel le nitrile de départ est le 2-méthyl-3-butènenitrile.

**7.** Procédé suivant la revendication 1, dans lequel l'intervalle de température est de 145°C à 200°C.